# EUROPEAN PATENT SPECIFICATION

(11) **EP 2 723 310 B1**
(45) Date of publication and mention of the grant of the patent: **07.12.2016**
(21) Application number: 12734800.1
(22) Date of filing: 22.06.2012
(51) Int. Cl.: A61K 8/49, A61K 8/73, A61Q 5/06

(54) **PROCESS FOR SEMI-PERMANENT STRAIGHTENING OF CURLY, FRIZZY OR WAVY HAIR USING SOLUTIONS CONTAINING CHITOSAN, ALLANTOIN AND GLYOXYLIC ACID DERIVATIVES COMBINED WITH HEAT.**
VERFAHREN ZUR SEMIPERMANENTEN AUSRICHTUNG VON GEKRÄUSELTEM, WUSCHELIGEM ODER GEWELLTEM HAAR MITHILFE VON LÖSUNGEN MIT CHITOSAN-, ALLANTOIN- UND GLYOXYLSÄUREDERIVATEN IN KOMBINATION MIT WÄRME
PROCÉDÉ DE DÉFRISAGE SEMI-PERMANENT DE CHEVEUX BOUCLÉS, FRISÉS OU ONDULÉS AU MOYEN DE SOLUTIONS CONTENANT DES DÉRIVÉS DE CHITOSANE, D'ALLANTOÏNE ET D'ACIDE GLYOXYLIQUE EN COMBINAISON AVEC DE LA CHALEUR

(30) Priority: 24.06.2011 IT AP20110009
(43) Date of publication of application: 30.04.2014
(73) Proprietor: D'Ottavi, Adele, 63048 Montemonaco (AP) (IT); Mannozzi, Alderano, 63100 Ascoli Piceno (AP) (IT)
(72) Inventor: D'OTTAVI , Adele, I-63048 Montemonaco (AP) (IT)
(74) Representative: Statti, Francesco
(86) International application number: PCT/EP2012/002649
(87) International publication number: WO 2012/175221

(56) References cited:
- EP-A2- 1 661 551
- WO-A1-2007/135299
- DE-A1- 19 853 842
- FR-A1- 2 865 210
- JP-A- 2005 194 261
- US-A1- 2003 143 173
- US-A1- 2005 136 017
- MUZARRELLI ET ALL: "N-(carboxymethylidene)chitosans and N-(carboxymethyl)chitosans : novel chelating polyampholytes obtained from chitosan glyoxylate", CARBOHYDRATE RESEARCH, vol. 107, 1982, pages 199-214, XP002670079,
- DATABASE WPI Week 197728 Thomson Scientific, London, GB; AN 1977-50012Y XP002670080, & SU 537 115 A (KAZAN KIROV CHEM TECHN) 17 January 1977 (1977-01-17)

## Description

### Field of invention

This invention relates to a process capable of straightening in a semi-permanent way curly, frizzy or wavy hair by using solutions containing chitosan, allantoin, and glyoxylic acid derivatives combined with heat.

### Background

At the state of the art, both the structure of human hair (hereinafter refereed to as *"hair fibre" or "keratinous fibre "*) and the substances used to modify its appearance as well as the hair-straightening techniques have been well described in numerous patent documents.

In patent WO 2007/135299 A1 there is a description of a process that consists of the application of aqueous solutions of alpha-hydroxy and/or alpha-ketone acids on hair and subsequent straightening using hair-straightening irons set to a temperature ranging between 110 and 250°C.

This patent describes and discloses only alpha-ketone acids of the general formula: 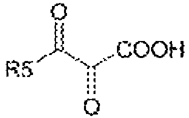 in which the R5 radical represents different functional groups, all different from the hydrogen atom (present on the contrary in glyoxylic acid).

Vice versa, the molecules that are the subject matter of this patent application, as far as carboxylic acids are concerned, have an aldehyde group (glyoxylic acid) 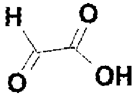 as well as an allantoin and/or chitosan group which are not included in the types of functional groups listed in the above mentioned previous patent application WO 2007/135299 A1.

Moreover, in this previous patent there was no description or disclosure of the imine derivatives (-N=C-) included in this patent application in which the carbon atom is bound to one hydrogen atom and to a carboxylic functional group (-COOH) according to the following formula:

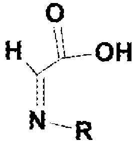

The use of alkanoic carboxylic acids of the general formula: 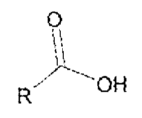 is described and disclosed in the previous patent US 2003/143173 A1, in which the functional group R is neither an aldehyde group nor an imine one, but an alkane, alkene or similar group.

Vice versa, this patent application describes and discloses carboxylic acid molecule in which the R group is composed of an imine group (-N=C-) which is part of the allantoin or chitosan molecule or of carboxylic acids, in which the R group is an aldehyde group (glyoxylic acid).

Patent EP 1661 551 A2 describes and discloses molecules in which there is a carbonylic functional group whose carbon atom is bound to two nitrogen atoms ("*derivative containing a C=O group bound to two nitrogen atoms* ") according to the following formulas: 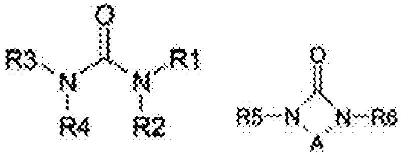 and molecules in which the carbon of an incline group is bound to two nitrogen atoms like in the following formula:

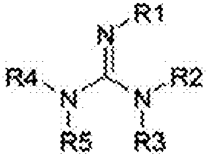

Patent US 2005/136017 A1 describes and discloses non-hydroxyfunctional imines (*"not belonging to the hydroxide family"*) in which the carbon of the imine group (-N=C=) is bound contemporarily to one nitrogen atom and to one carbon (or nitrogen) atom according to the following formulas:

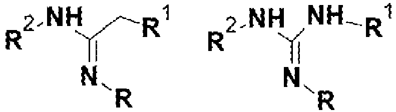

Vice versa, in the molecules of the imine groups (-N=C-) described and disclosed in this patent application, the carbon atom is bound to one hydrogen atom and to one carbon atom belonging to a carboxylic functional group (-COOH) according to the following formula:

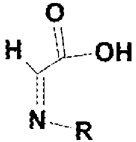

In general, the action mechanisms that regulate the straightening effect of wavy and/or frizzy hair involve the breakage of cysteine disulphide bridges with their subsequent rearrangement after the physicomechanical modification of the hair structure.

An alternative to the breakage of the cysteine disulphide bridges is represented by the use of glyoxylic acid, which was described in the previous patents US 4752467 and SU 537 115 A.

Glyoxylic acid is an extremely particular carboxylic acid inasmuch that its short carbonious chain (only 2 carbon atoms) presents a carboxylic functional group and an aldehyde functional group. The action mechanism of the aldehyde group is based on the new formation of methylene bridges between the amino groups of the amino acids that constitute the keratinous fibres by means of acidity and heat as described in the article published in the journal "*Cosmetiscope*" summer 2011 of the New York Society of Cosmetic Chemists (www.nyscc.org).

The previous patents WO 2007/135299 and FR 2901472 described that high temperature was used to influence the shape of hair fibres together with the presence on the fibres themselves of one or more alpha-hydroxy and/or alpha-ketone acids. The above mentioned documents do not disclose the use of glyoxylic acid among the substances described and disclosed in this patent application, as this substance cannot be classified either as an alpha-hydroxy acid or as an alpha-ketone acid.

According to the international nomenclature established by the International Union of Pure and Applied Chemistry (IUPAC) "Compendium of Chemical Terminology" 2nd Edition (1997), glyoxylic acid must be classified as an oxocarboxylic acid.

In the previous patent SU 537 115 A there is a description of a glyoxylic acid, ethanol and water based solution capable of straightening keratinous fibres. The purpose of the solution is to reduce the toxicity of formaldehyde, the toxicity of which for the health of those regularly exposed to its vapours was already well known back in 1977.

Patent SU 537115 A relates exclusively to a solution containing glyoxylic acid, methanol and water.

The molecules that are the subject matter of this patent application are related to carboxylic acids in which the R group is composed of an imine group (-N=C-) belonging either to the allantoin or chitosan molecule.

Chitosan has also been described in numerous patent documents regarding its application as a conditioning agent of hair fibres (e.g. US 4752467 and US 6248313 B1).

The formation of carboxyimine chitosan derivatives is also well known and has been well described: "N-(carboxymethylidene) chitosan and N-(carboxymethyl)chitosan: novel chelating polyampholytes obtained from chitosan glyoxylate" published in Carbohydrate research Vo. 107 (1982) 199-214 by Professor Riccardo Muzzarelli.

The carboxyimine chitosan derivative denominated by Professor Muzzarelli 'N-(carboxymethylidene)chitosan' is easily obtained by simply mixing chitosan into an aqueous solution of glyoxylic acid. The reaction takes place in equimolecular ratios as a glyoxylic acid molecule reacts with a monomer of the chitosan polymeric chain (1:1 ratio) according to the formula illustrated in the annexed definitions. The carboxyimine group on this molecule is a particular type of Schiff base in which the carbon of the group (-N=C-) is bound to a carboxylic group (-COOH).

Allantoin is a substance which is widely used in cosmetics and there are an incredible number of patents that state its various applications, most of which are summarised in EP 2 172 106 A1. Allantoin has poor solubility in water (a maximum of 0.4%), a factor which limits its application in cosmetic products. Its solubility can be increased by mixing it together with urea derivatives as described in US 2008081052 A1 and US 2008057002 A1.

Already in 1987 in patent JP 1096105 A there was a description of the reaction between allantoin and glyoxylic acid leading to the formation of a new substance with various applications in cosmetics.

In patent DE 19853842 A1 this reaction was generically cited by the inventor as one of the potential applications in the cosmetics industry.

Allantoin when dispersed in water dissolves completely when it comes into contact with an aqueous solution of glyoxylic acid thus forming a new substance which in the two previously cited documents is generically called "condensate". Considering that the allantoin molecule contains a free amino group (-NH2), similar to that or chitosan, it is fair to believe that following their binding the relative carboxyimine derivative N-(carboxymethylidene)allantoin is formed with the same mechanism advanced by the previously mentioned Professor Muzzarelli.

Also in this case, as in the case of chitosan, the reaction takes place in equimolecular ratios as one molecule of glyoxylic acid reacts with one molecule of allantoin (1:1 ratio) according to the formula illustrated in the annexed definitions.

### Disclosure of invention

The purpose of this invention is to demonstrate that carboxyimine chitosan and allantoin derivatives (obtained through the reaction between glyoxylic acid and chitosan or Allantoin) are capable of greatly improving the semi-permanent deformation of keratinous fibres compared to the exclusive use of glyoxylic acid.

The other purpose of this invention is to achieve hair fibres characterised by a reduction in volume and a decrease in the number of curves, as shown in the tables submitted together with this patent application.

### Detailed description

These and other purposes are achieved through the present invention that comprises the following phases:
a) the application on the hair fibres of solutions containing carboxyimine chitosan and/or allantoin derivatives (obtained through the reaction between glyoxylic acid and respectively chitosan and allantoin);
b) contact of the solution with hair for a period of time ranging between 30 and 120 minutes;
c) hair drying using a hairdryer;
d) hair-straightening using a hair-straightening iron according to known systems whose temperature above 170°C varies according to the hair type.

Further characteristics of the invention will be explained more clearly in the detailed description below which refers to an application stated purely as an example and therefore not restrictive.

By way of example, listed below are some examples of solutions used in the conditioning of hair fibres:

### Example of solution 1:

a) disperse 1 gram of chitosan in 97 grams of deionized water;
b) dissolve 1 gram of crystalline glyoxylic acid in 1 gram of water;
c) add solution b) to solution a);
d) agitate at a temperature of 35-40°C until complete dissolution.

The solution obtained contains around 1 gram of N-(carboxymethylidene)chitosan.

### Example of solution 2:

a) disperse 1 gram of allantoin in 97 grams of deionised water;
b) dissolve 1 gram of crystalline glyoxylic acid in 1 gram of water;
c) add solution b) to solution a);
d) agitate at a temperature of 50-55°C until complete dissolution.

The solution obtained, considering the reaction between allantoin and glyoxylic acid in a 1:1 ratio, contains around 1 gram of N-(carboxymethylidene)allantoin.

### Example of solution 3:

starting materials:
   - chitosan solution consisting of 1 gram of chitosan dispersed in 96 grams of deionised water;
   - glyoxylic acid solution consisting of 2 grams of crystalline glyoxylic acid dissolved in 2 grams of water;
   - 1 gram of allantoin;
procedure:
   a) add glyoxylic acid solution to chitosan solution and agitate at a temperature of 35-40°C until complete dissolution;
   b) add 1 gram of allantoin.

The solution obtained contains around 1 gram of N-(carboxymethylidene)chitosan and around 1 gram of N-(carboxymethylidene)allantoin.

### Example of solution 4:

starting materials:
   - chitosan solution consisting of 1 gram of chitosan dispersed in 94.5 grams of deionized water;
   - glyoxylic acid solution consisting of 2 grams of crystalline glyoxylic acid dissolved in 2 grams of water;
   - 1 gram of allantoin;
   - 1 gram of Cetrimonium chloride solution 30%;
   - 0.5 grams of citric acid;
procedure:
   a) add glyoxylic acid solution to chitosan solution and agitate at a temperature of 35-40°C until complete dissolution;
   b) add 1 gram of allantoin and agitate until complete dissolution;
   c) add 1 gram of Cetrimonium chloride solution 30%;
   d) add 0.5 grams of citric acid and agitate until complete dissolution.

The solution obtained contains around 1 gram of N-(carboxymethylidene)chitosan and around 1 gram of N-(carboxymethylidene)allantoin, as well as ingredients commonly used in the cosmetics industry.

The above described solutions were applied as described below on hair that had been previously washed according to common use.

The performance of such solutions in terms of straightening efficiency and volume reduction was compared with glyoxylic acid solutions in concentrations of 1% and 2% in water as illustrated in the annexed tables.

These solutions were then applied on hair using a hard-bristle brush. The hair was covered with a plastic cap in order to reduce water evaporation and left in contact with the above mentioned solutions for 30, 60, 120 minute, after which time the hair was dried with a hairdryer according to common use. Once dried, the hair underwent straightening using a hair-straightening iron heated to a temperature higher than 170°C (210°C or 230°C in the cases illustrated in Tables 1 to 5 according to the hair type).

Finally, the hair was washed with water and shampoo and dried several times checking at each wash the reduction in volume and the presence of curves.

The results of the above tests are shown in the following Tables 1 to 5. The modification of the shape of the hair fibres (reduction in volume and number of curves) using the above mentioned solutions proved to be stable and efficient in subsequent washings as compared to using only glyoxylic acid. In particular, in the case of solutions 3 and 4, for 'afro' type hair, straightening and reduction in volume proved to be stable over time up to 8 shampoos, rinsings and drying according to common use of hair shampoos, whilst when only glyoxylic acid was used, this result was lost after just the first 4 shampoos.

In natural curly Caucasian type hair, the straightening effect and volume reduction resulted higher than that achieved with glyoxylic acid always exceeding 8 shampoos and in certain cases reaching 16 shampoos.

In 'afro' type hair a better performance of solutions 3 and 4, in terms of straightening and volume reduction, was observed when these solutions remained in contact with hair for a longer period of time (up to 120 minutes) as shown in Table 5.

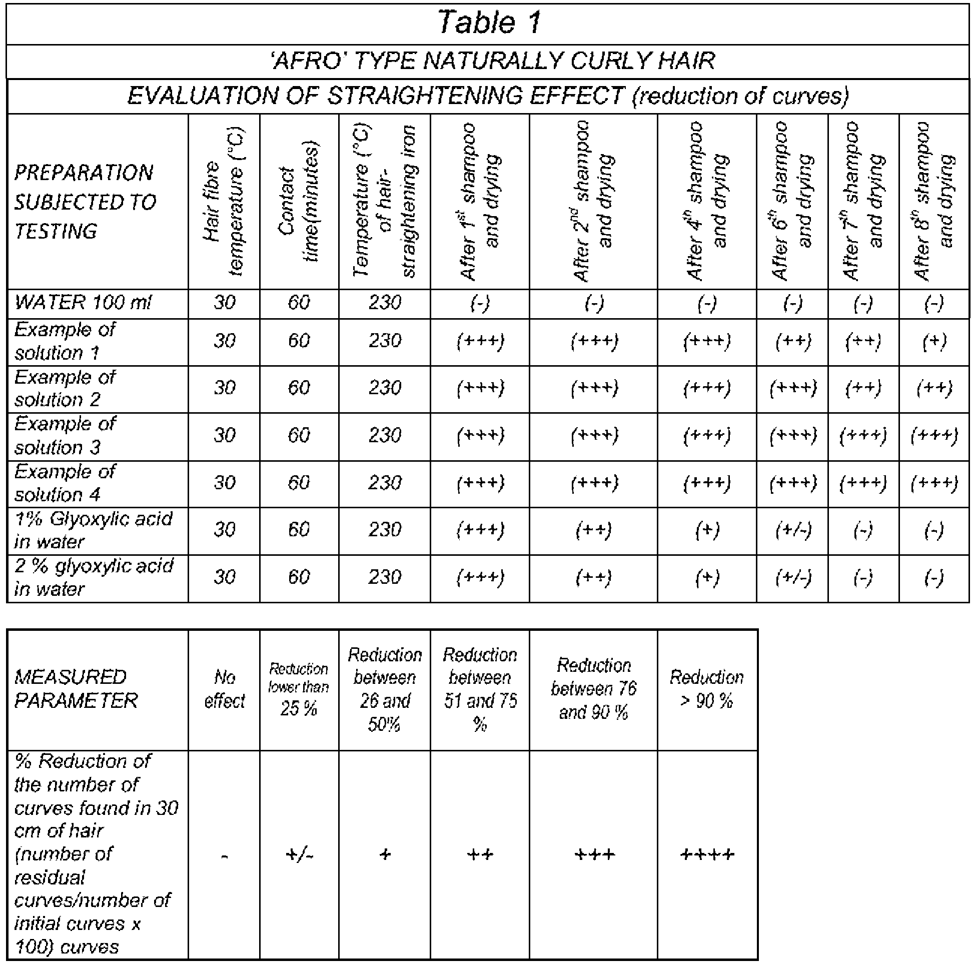

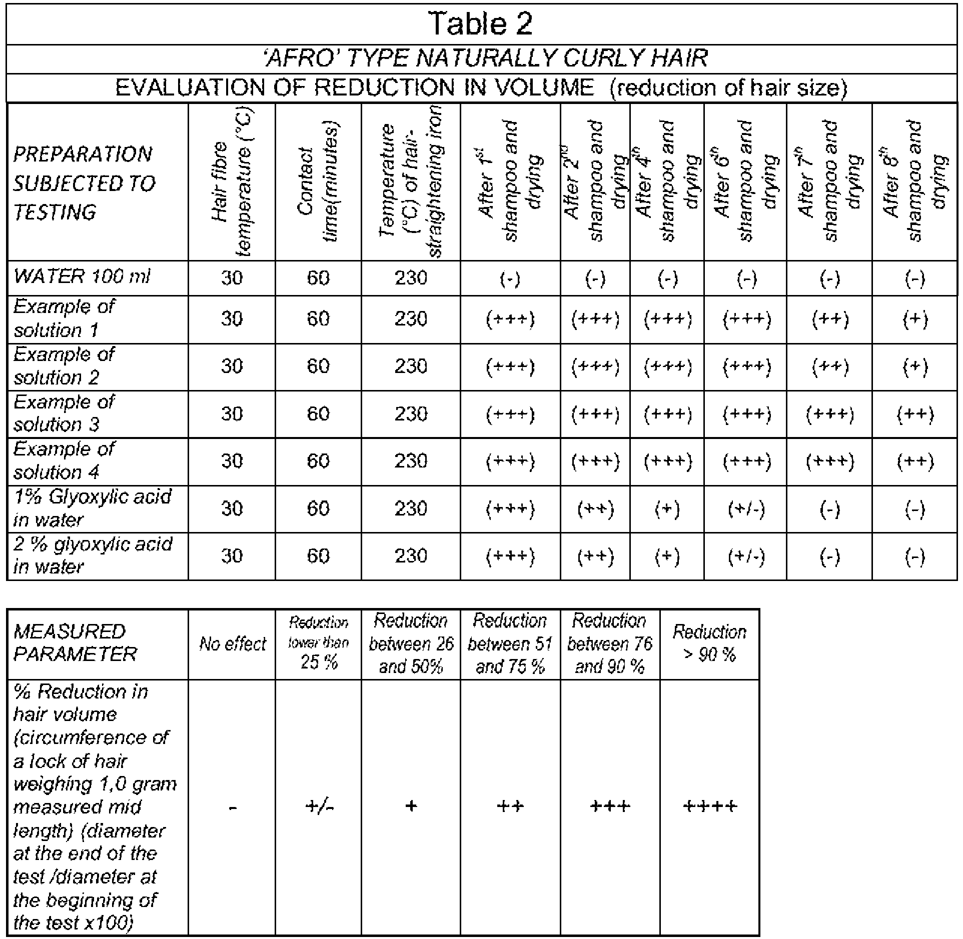

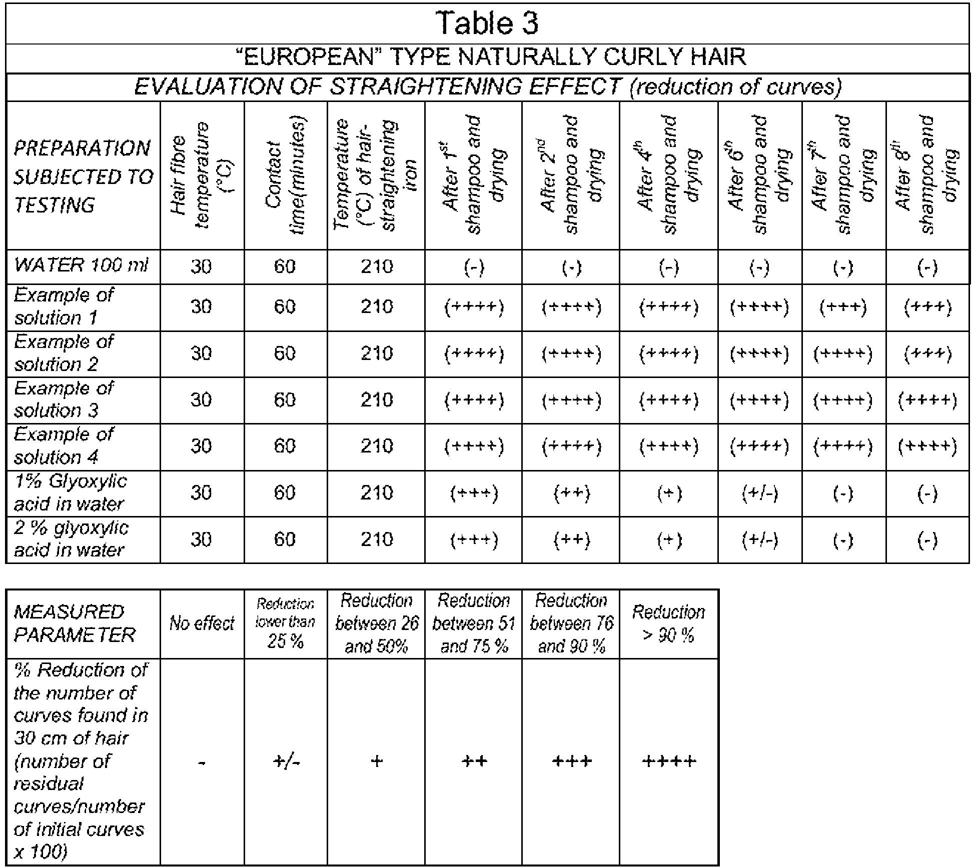

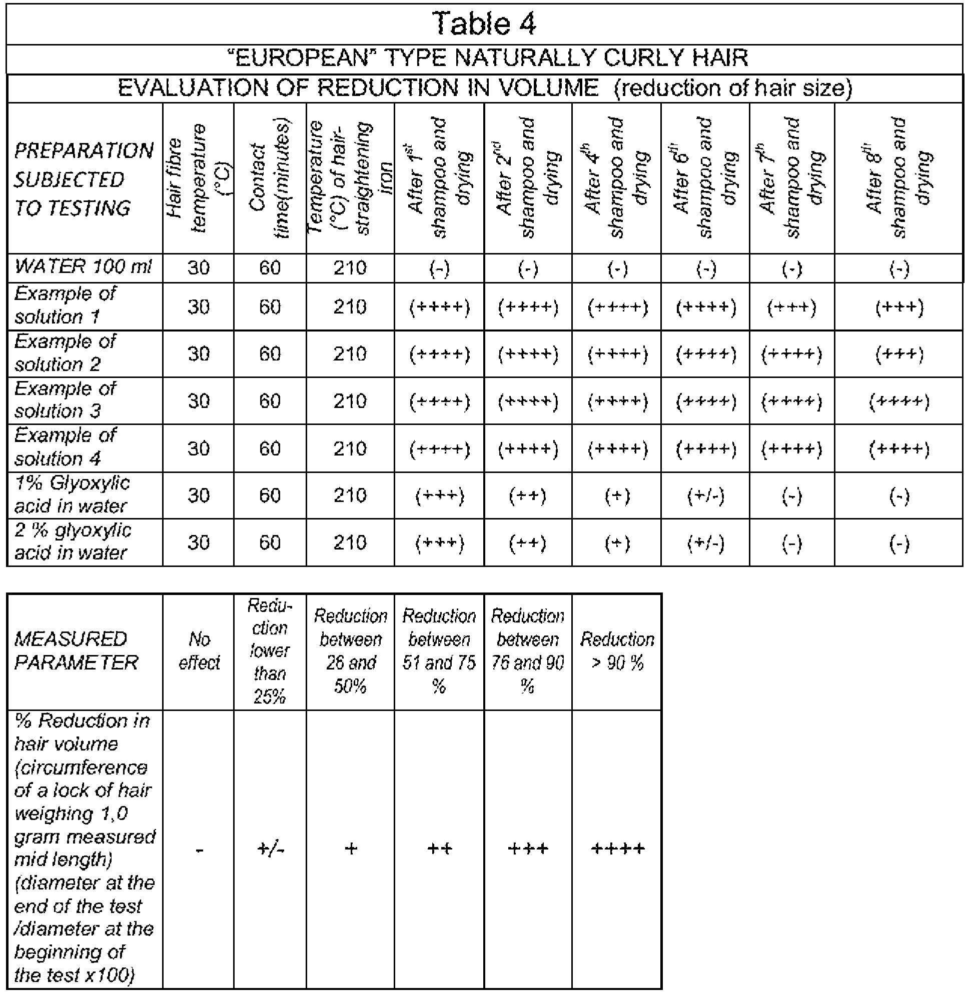

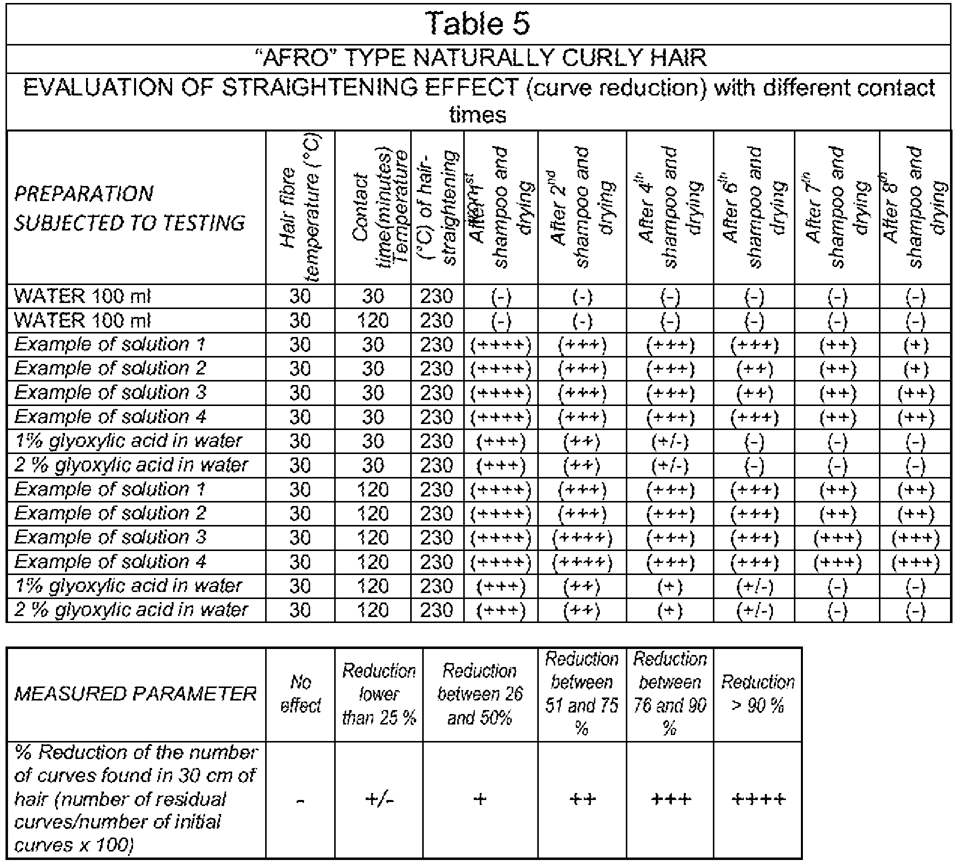

### DEFINITIONS

**Semi-permanent treatment:** the treatment of curly or frizzy hair in order to straighten and maintain straightening for at least 8 shampoos without using substances capable of reacting with cysteine disulphide bridges.

**Permanent treatment:** a treatment to make hair curly or straight for at least 8 shampoos by means of an oxidation-reduction process of the cysteine disulphide bridges contained in the hair fibres or through hydrolysis of the peptidic bonds with alkali.

**Carboxyimine derivative:** an organic substance containing the carboxyimine functional group (-N=C-COOH), indicated by -*N-(carboxymethylidene).*

**Hair-straightening iron:** an electrical device composed of two heating plates coated in different materials between which one lock of hair at a time is placed.

**N-(carboxymethylidene)chitosan:** a carboxyimine chitosan derivative resulting from the reaction between glyoxylic acid and chitosan according to the following reaction:

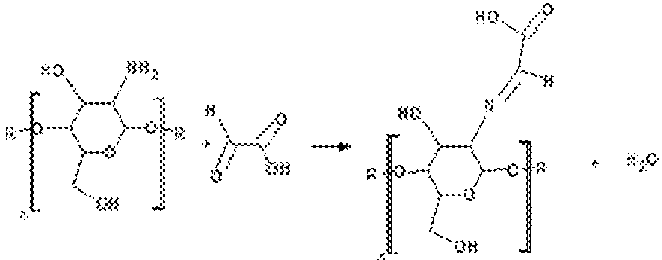

**N-(carboxymethylidene)allantoin:** carboxyimine allantoin derivative resulting from the reaction between glyoxylic acid and allantoin according to the following reaction:

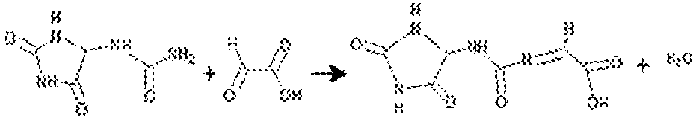

## Claims

1. A process for semi-permanent straightening of human hair comprising:
a) the use of a solution containing N-(carboxymethylidene) chitosan;
b) contact of the solution with hair for a length of time between 30 and 120 minutes;
c) hair drying with a hairdryer;
d) hair-straightening using a hair-straightening iron set to a temperature higher than 170°C according to the hair type.

2. A process for semi-permanent straightening of human hair comprising:
e) the use of a solution containing N-(carboxymethylidene) allantoin;
f) contact of the solution with hair for a length of time between 30 and 120 minutes;
g) hair drying with a hairdryer;
h) hair-straightening using a hair-straightening iron set to a temperature higher than 170°C according to the hair type.

3. A process for semi-permanent straightening of human hair comprising:
i) the use of a solution containing N-(carboxylmethylidene) chitosan and N-(carboxymethylidene)allantoin:
j) contact of the solution with hair for a length of time between 30 and 120 minutes;
k) hair drying with a hairdryer;
l) hair-straightening using a hair-straightening iron set to a temperature higher than 170°C according to the hair type.

## Patentansprüche

1. Verfahren zum semipermanenten Ausrichten von menschlichem Haar, **gekennzeichnet durch**:
a) das Verwenden von einer Lösung, die N-(Carboxymethyliden) Chitosan enthält;
b) Kontakt der Lösung mit dem Haar für eine Zeitspanne zwischen 30 und 120 Minuten;
c) Trocknen des Haars mit einem Fön;
d) Haarausrichten **durch** Verwenden von einem Glätteisen mit einer Temperatur über 170 °C entsprechend dem Haartyp.

2. Verfahren zum semipermanenten Ausrichten von menschlichem Haar, **gekennzeichnet durch**:
e) das Verwenden von einer Lösung, die N-(Carboxymethyliden) Allantoin enthält;
f) Kontakt der Lösung mit dem Haar für eine Zeitspanne zwischen 30 und 120 Minuten;
g) Trocknen des Haars mit einem Fön;
h) Haarausrichten **durch** Verwenden von einem Glätteisen mit einer Temperatur über 170 °C entsprechend dem Haartyp.

3. Verfahren zum semipermanenten Ausrichten von menschlichem Haar, **gekennzeichnet durch**:
i) das Verwenden von einer Lösung, die N-(Carboxymethyliden) Chitosan und N-(Carboxymethyliden) Allantoin enthält;
j) Kontakt der Lösung mit dem Haar für eine Zeitspanne zwischen 30 und 120 Minuten;
k) Trocknen des Haars mit einem Fön;
l) Haarausrichten **durch** Verwenden von einem Glätteisen mit einer Temperatur über 170°C entsprechend dem Haartyp.

## Revendications

1. Procédé de défrisage semi-permanent des cheveux humains, comprenant:
a) l'utilisation d'une solution contenant du N-(carboxyméthylidène) chitosane;
b) le contact de la solution avec les cheveux pendant un laps de temps compris entre 30 et 120 minutes;
c) le séchage des cheveux au moyen d'un sèche-cheveux;
d) le défrisage des cheveux en utilisant un jeu de fers à lisser, à une température supérieure à 170°C en fonction du type de cheveux.

2. Procédé de défrisage semi-permanent des cheveux humains, comprenant:
e) l'utilisation d'une solution contenant de la N-(carboxyméthylidène) allantoïne;
f) le contact de la solution avec les cheveux pendant un laps de temps compris entre 30 et 120 minutes;
g) le séchage des cheveux au moyen d'un sèche-cheveux;
h) le défrisage des cheveux en utilisant un jeu de fers à lisser, à une température supérieure à 170°C en fonction du type de cheveux.

3. Procédé de défrisage semi-permanent des cheveux humains, comprenant:
i) l'utilisation d'une solution contenant du N-(carboxyméthylidène) chitosane et de la N-(carboxyméthylidène) allantoïne;
j) le contact de la solution avec les cheveux pendant un laps de temps compris entre 30 et 120 minutes;
k) le séchage des cheveux au moyen d'un sèche-cheveux;
1) le défrisage des cheveux en utilisant un jeu de fers à lisser, à une température supérieure à 170°C en fonction du type de cheveux.
